# EUROPEAN PATENT APPLICATION

(11) **EP 4 613 739 A1**
(43) Date of publication of application: **10.09.2025**
(21) Application number: 23912178.3
(22) Date of filing: 26.12.2023
(51) Int. Cl.: C07C 321/14, C07D 319/06

(54) **TRISULFIDE COMPOUND**

(30) Priority: 27.12.2022 JP 2022210344
(71) Applicant: Kyowa Pharma Chemical Co., Ltd., Takaoka-shi, Toyama 933-8511 (JP)
(72) Inventor: TOMONAGA, Shoichiro, Takaoka-shi, Toyama 933-8511 (JP); ISHIMARU, Hiroaki, Takaoka-shi, Toyama 933-8511 (JP); OHSHIMA, Etsuo, Tokyo 164-0001 (JP)
(74) Representative: Vossius & Partner Patentanwälte Rechtsanwälte mbB
(86) International application number: PCT/JP2023/046749
(87) International publication number: WO 2024/143400

(57) **Abstract**

Disclosed is a compound represented by formula (1) or a salt thereof. (In the formula: R¹⁰ is a hydrogen atom or the like; R¹¹ is a hydrogen atom or the like; and h and i are each independently 0 or 1.)

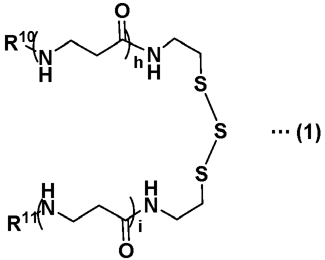

## Description

### Technical Field

The present invention relates to trisulfide compounds.

### Background Art

Compounds containing a covalent bond structure of three consecutive sulfur atoms are called trisulfide compounds. Trisulfide compounds are expected to have various physiological activities due to their redox capability, which depends on the possible valence states of the constituent sulfur atoms. As a method for producing trisulfide compounds, the method described in Patent Literature 1 is known.

As a trisulfide compound, for example, pantethine trisulfide is known (Patent Literature 2). Patent Literature 2 discloses that pantethine trisulfide has a hydrogen sulfide elimination effect, and the usefulness of pantethine trisulfide as a pharmaceutical is expected.

### Citation List

### Patent Literature

[Patent Literature 1] WO2021/200487
[Patent Literature 2] WO2022/045052

### Summary of Invention

### Technical Problem

An object of the present invention is to provide a novel trisulfide compound.

### Solution to Problem

The present invention relates, for example, to the following [1] to [10].
[1] A compound represented by the Formula (1) or a salt thereof: 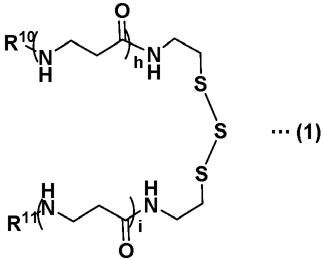
   wherein R¹⁰ is a hydrogen atom or a group represented by 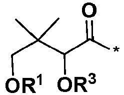 wherein * is a bond, and
   R¹¹ is a hydrogen atom or a group represented by 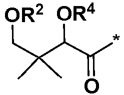 wherein * is a bond, with a proviso that R¹⁰ and R¹¹ are not hydrogen atoms at the same time,
   wherein R¹, R², R³, and R⁴ are each independently a hydrogen atom, a C₁₋₉ alkyl group, or a C₁₋₅ acyl group, and R¹ and R³ may together form a group represented by the Formula (2): 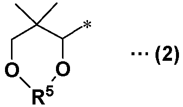 wherein R⁵ is a methylene group optionally having a C₁₋₄ alkyl group as a substituent, and * is a bond, or a carbonyl group, R² and R⁴ may together form a group represented by the Formula (2): 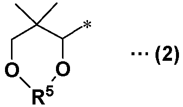 wherein R⁵ is a methylene group optionally having a C₁₋₄ alkyl group as a substituent, and * is a bond, or a carbonyl group, with a proviso that at least one of R¹, R², R³, or R⁴ is a C₁₋₉ alkyl group or a C₁₋₅ acyl group, and h and i are each independently 0 or 1.
[2] The compound or salt thereof according to [1], wherein R¹, R², R³, and R⁴ are each independently a hydrogen atom or a C₄ alkyl group, and at least one of R¹, R², R³, or R⁴ is a C₁₋₄ alkyl group.
[3] The compound or salt thereof according to [1], wherein R¹, R², R³, and R⁴ are each independently a hydrogen atom or a C₁₋₅ acyl group, and at least one of R¹, R², R³, or R⁴ is a C₁₋₅ acyl group.
[4] The compound or salt thereof according to [1], wherein R¹ and R³ together form a group represented by Formula (2) or a carbonyl group, and R² and R⁴ together form a group represented by Formula (2) or a carbonyl group.
[5] The compound or salt thereof according to any one of [1] to [4], wherein h and i are 0.
[6] A compound represented by the Formula (1a) or a salt thereof: 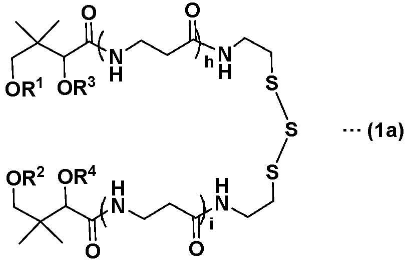 wherein R¹, R², R³, and R⁴ are each independently a hydrogen atom, a C₁₋₉ alkyl group, or a C₁₋₅ acyl group, R¹ and R³ may together form a group represented by the Formula (2): 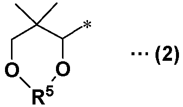 wherein R⁵ is a methylene group optionally having a C₁₋₄ alkyl group as a substituent, and * is a bond, or a carbonyl group, R² and R⁴ may together form a group represented by the Formula (2): 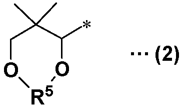 wherein R⁵ is a methylene group optionally having a C₁₋₄ alkyl group as a substituent, and * is a bond, or a carbonyl group, with a proviso that at least one of R¹, R², R³, or R⁴ is a C₁₋₉ alkyl group or a C₁₋₅ acyl group, and h and i are each independently 0 or 1.
[7] The compound or salt thereof according to [6], wherein R¹, R², R³, and R⁴ are each independently a hydrogen atom or a C₁₋₄ alkyl group, and at least one of R¹, R², R³, or R⁴ is a C₁₋₄ alkyl group.
[8] The compound or salt thereof according to [6], wherein R¹, R², R³, and R⁴ are each independently a hydrogen atom or a C₁₋₅ acyl group, and at least one of R¹, R², R³, or R⁴ is a C₁₋₅ acyl group.
[9] The compound or salt thereof according to [6], wherein R¹ and R³ together form a group represented by Formula (2) or a carbonyl group, and R² and R⁴ together form a group represented by Formula (2) or a carbonyl group.
[10] The compound or salt thereof according to any one of [6] to [9], wherein h and i are 0.

### Advantageous Effects of Invention

According to the present invention, it is possible to provide a novel trisulfide compound. Pantethine trisulfide is expected to be a compound excellent in water solubility and stability, but in some cases, a compound with higher lipophilicity (a compound with a better balance of water solubility and lipophilicity) is more suitable as a pharmaceutical. The above compound (1) is expected to be a compound having a better balance of water solubility and lipophilicity while having stability equal to or greater than that of pantethine trisulfide. The above compound (1) can be said to be a compound in which the hydroxyl group(s) of pantethine trisulfide is(are) modified or protected. By modifying or protecting the hydroxyl group(s), the number of hydrogen bonding present in the molecule of pantethine trisulfide is reduced, which is considered to improve lipophilicity. In addition, when at least one of h and i in compound (1) is 0, the number of amide bonds which participate in hydrogen bonding is reduced, which is also considered to improve lipophilicity. Furthermore, in these cases, the molecular weight is reduced, which is considered to improve bioavailability.

### Brief Description of Drawings

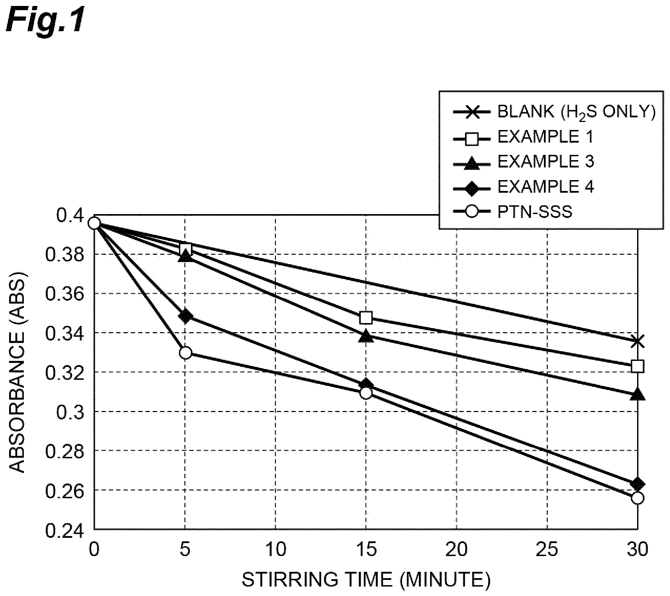

Figure 1 is a graph showing the test results of hydrogen sulfide trapping ability (hydrogen sulfide elimination effect) in Test Example 1.

### Description of Embodiments

A compound according to one embodiment of the present invention is a compound represented by the above Formula (1). A compound according to another embodiment of the present invention is a compound represented by the above Formula (1a). In Formulae (1) and (1a), R¹, R², R³, and R⁴ are each independently a hydrogen atom, a C₁₋₉ alkyl group, or a C₁₋₅ acyl group, and at least one of R¹, R², R³, or R⁴ is a C₁₋₉ alkyl group or a C₁₋₅ acyl group. R¹, R², R³, and R⁴ may each independently be a hydrogen atom or a C₁₋₄ alkyl group, and at least one of R¹, R², R³, or R⁴ may be a C₁₋₄ alkyl group. R¹, R², R³, and R⁴ may each independently be a hydrogen atom or a C₁₋₅ acyl group, and at least one of R¹, R², R³, or R⁴ may be a C₁₋₅ acyl group. In Formulae (1) and (1a), R¹ and R² may be the same, R³ and R⁴ may be the same, R¹, R², and R³ may be the same, R², R³, and R⁴ may be the same, or all of R¹, R², R³, and R⁴ may be the same.

Examples of the C₁₋₄ alkyl group in Formulae (1) and (1a) include methyl, ethyl, n-propyl, isopropyl, cyclopropyl, n-butyl, isobutyl, sec-butyl, tert-butyl, and cyclobutyl. Examples of the C₁₋₅ acyl group in Formulae (1) and (1a) include formyl (methanoyl), acetyl (ethanoyl), n-propanoyl, isopropanoyl, cyclopropanoyl, n-butanoyl, isobutanoyl, sec-butanoyl, tert-butanoyl, and cyclobutanoyl. From the viewpoint of lowering the molecular weight of compound (1) and compound (1a), the C₁₋₄ alkyl group is preferably methyl or ethyl, and the C₁₋₅ acyl group is preferably formyl or acetyl.

Examples of combinations of R¹, R², R³, and R⁴ are shown in Table 1. The methyl group in Table 1 (when multiple methyl groups are present in the same molecule, each methyl group is independently considered) may be replaced with an ethyl, n-propyl, isopropyl, cyclopropyl, n-butyl, isobutyl, sec-butyl, tert-butyl, or cyclobutyl group.

**[Table 1]**

| R¹ | R² | R³ | R⁴ |
|---|---|---|---|
| methyl | hydrogen | hydrogen | hydrogen |
| methyl | methyl | hydrogen | hydrogen |
| methyl | methyl | methyl | hydrogen |
| methyl | methyl | methyl | methyl |
| hydrogen | hydrogen | methyl | methyl |
| methyl | hydrogen | methyl | hydrogen |
| methyl | hydrogen | methyl | methyl |

Another example of combinations of R¹, R², R³, and R⁴ is shown in Table 2. The formyl group in Table 2 (when multiple formyl groups are present in the same molecule, each formyl group is independently considered) may be replaced with an acetyl, propanoyl, butanoyl, or pentanoyl group. The butanoyl and pentanoyl groups may be linear, branched, or cyclic.

**[Table 2]**

| R¹ | R² | R³ | R⁴ |
|---|---|---|---|
| formyl | hydrogen | hydrogen | hydrogen |
| formyl | formyl | hydrogen | hydrogen |
| formyl | formyl | formyl | hydrogen |
| formyl | formyl | formyl | formyl |
| hydrogen | hydrogen | formyl | formyl |
| formyl | hydrogen | formyl | hydrogen |
| formyl | hydrogen | formyl | formyl |

An example of combinations of R¹ and R³, and R² and R⁴, which together form a group represented by Formula (2) or a carbonyl group, is shown in Table 3. The methyl group in Table 3 (when multiple methyl groups are present in the same molecule, each methyl group is independently considered) may be replaced with an ethyl, n-propyl, isopropyl, cyclopropyl, n-butyl, isobutyl, sec-butyl, tert-butyl, or cyclobutyl group. The number of substituents such as methyl groups may be one or two.

**[Table 3]**

| R¹ and R³ | R² and R⁴ |
|---|---|
| methylene | methylene |
| methylene having methyl as substituent | methylene |
| methylene having methyl as substituent | methylene having methyl as substituent |
| carbonyl | carbonyl |

In Formulae (1) and (1a), h and i are each independently 0 or 1. h and i may be different or the same. When at least one of h and i is 0, the lipophilicity of compound (1) and compound (1a) is considered to be further improved, and the balance of water solubility and lipophilicity is considered to be excellent. In addition, in this case, since the molecular weight of compound (1) and compound (1a) can be reduced, bioavailability is considered to be improved. These tendencies are considered to be more pronounced when both h and i are 0.

The molecular weight of compound (1) and compound (1a) and salts thereof may be 800 or less, 750 or less, or 700 or less, and preferably 600 or less, 500 or less, or 450 or less. The molecular weight of compound (1) and compound (1a) and salts thereof may be 300 or more, 350 or more, or 400 or more. When the molecular weight of compound (1) and compound (1a) and salts thereof is within these ranges, bioavailability is considered to be further improved.

The ClogP of compound (1) and compound (1a) and salts thereof may be -1 or more, -0.5 or more, 0 or more, 0.5 or more, 1 or more, or 1.5 or more. The ClogP of compound (1) and compound (1a) and salts thereof may be 5 or less, 4.5 or less, 4 or less, 3.5 or less, 3 or less, or 2.5 or less. When the ClogP of compound (1) and compound (1a) and salts thereof is within these ranges, the balance of water solubility and lipophilicity of compound (1) and compound (1a) and salts thereof is considered to be more excellent. ClogP is a partition coefficient calculated by computer and can be determined according to the principles described in "CLOGP Reference Manual Daylight Version 4.9 (Release date: August 1, 2011, https://www.daylight.com/dayhtml/doc/clogp/)".

The salt of compound (1) and compound (1a) may be any pharmacologically acceptable salt, such as a salt with an alkali metal such as sodium or potassium; a salt with an alkaline earth metal such as calcium or magnesium; an ammonium salt, an inorganic acid salt such as hydrochloride; or an organic acid salt such as acetate. When compound (1) or compound (1a) is obtained as a free form, it can be converted to a salt by a conventional method. When compound (1) or compound (1a) is obtained as a salt, it can also be converted to a free form by a conventional method.

The compounds described herein may have four optical isomers: R,R-form, R,S-form, S,R-form, and S,S-form. The compound described herein may be replaced with its optical isomer or a mixture (*e.g*., racemate) comprising the optical isomers in any ratio. For example, compound (1a) may be an optical isomer represented by the following Formula (1-1), an optical isomer represented by the following Formula (1-2), an optical isomer represented by the following Formula (1-3), or an optical isomer represented by the following Formula (1-4), or may be replaced with a mixture (*e.g*., racemate) comprising these optical isomers in any ratio.

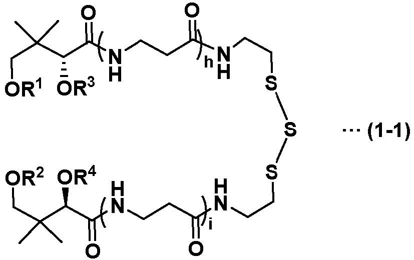

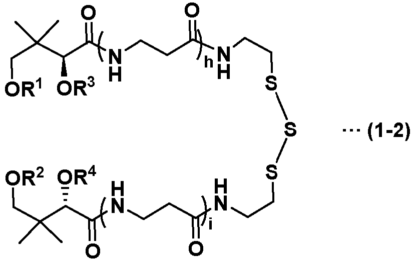

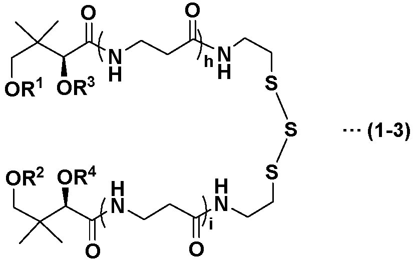

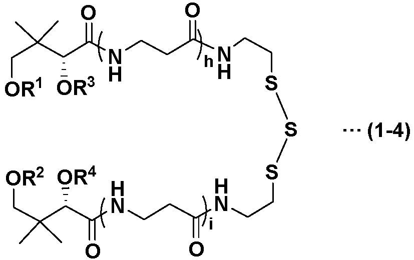

Compound (1) and compound (1a) and salts thereof can be formulated into a pharmaceutical composition by adding a pharmacologically acceptable additive as necessary. The pharmaceutical composition comprising compound (1) or compound (1a) or a salt thereof can be used for the prevention and treatment of pantothenic acid deficiency, similarly to pantethine, when at least one of h, i, j, or k is 1.

The pharmaceutical composition comprising compound (1) or compound (1a) or a salt thereof can be formulated, for example, as a parenteral or oral preparation. Examples of parenteral preparations include injections, nasal drops, eye drops, sublingual agents, transdermal agents (topical agents, patches, etc.), pulmonary agents (inhalants), nasal agents (inhalants), enteral agents (suppositories), and vaginal agents (vaginal suppositories). Examples of injections include subcutaneous injections, intramuscular injections, intravenous injections, intraperitoneal injections, intra-articular injections, and intraocular injections. Examples of oral preparations include tablets, granules, fine granules, powders, and capsules. Examples of additives include stabilizers such as saccharides (sucrose, trehalose, maltose, lactose, etc.), sugar alcohols (sorbitol, etc.), amino acids (L-arginine, etc.), water-soluble polymers (HES (hydroxyethyl starch), PVP (polyvinylpyrrolidone), etc.), nonionic surfactants (polysorbate, poloxamer, etc.), pH adjusters such as sodium phosphate buffer, histidine buffer, isotonic agents such as sodium chloride, and excipients such as mannitol, glycine, sodium chloride, and sucrose.

The above injection may be freeze-dried by a conventional method to provide a freeze-dried preparation for reconstitution at the time of use. The injection can be prepared by a method typically used in pharmaceutical manufacturing. Specifically, for example, water is placed in a container in an environment maintained at a constant temperature of 5 to 25°C, and while gently stirring, the previously weighed compound (1) or compound (1a) or a salt thereof and an additive are added. The pH is adjusted to the desired value, and the solution is sterilized by filtration or the like. The sterilized solution is filled into a container such as a glass vial and sealed with a rubber stopper or the like. When preparing a freeze-dried preparation, the obtained liquid preparation of the present invention may be freeze-dried by a known method. By preparing a freeze-dried preparation, compound (1) or compound (1a) or a salt thereof can be stably maintained for a long period.

Compound (1a) according to one embodiment of the present invention can be produced by:
(A) modifying at least one hydroxyl group of a trisulfide compound with a C₁₋₄ alkyl group or a C₁₋₅ acyl group, or protecting at least one pair of diols of the trisulfide compound by cyclic ketalization;
(B-1) modifying at least one hydroxyl group of a disulfide compound with a C₁₋₄ alkyl group or a C₁₋₅ acyl group, or protecting at least one pair of diols of the disulfide compound by cyclic ketalization, oxidizing the modified or protected disulfide compound with an oxidizing agent to obtain a sulfoxide compound, and trisulfidizing the obtained sulfoxide compound; or oxidizing the disulfide compound with an oxidizing agent to obtain a sulfoxide compound, modifying at least one hydroxyl group of the obtained sulfoxide compound with a C₁₋₄ alkyl group or a C₁₋₅ acyl group, or protecting at least one pair of diols of the sulfoxide compound by cyclic ketalization, and trisulfidizing the modified or protected sulfoxide compound; or
(B-2) modifying at least one hydroxyl group of a thiol compound with a C₁₋₄ alkyl group or a C₁₋₅ acyl group, or protecting at least one pair of diols of the thiol compound by cyclic ketalization, oxidizing the modified or protected thiol compound with an oxidizing agent to obtain a sulfoxide compound, and trisulfidizing the obtained sulfoxide compound; or oxidizing the thiol compound with an oxidizing agent to obtain a sulfoxide compound, modifying at least one hydroxyl group of the obtained sulfoxide compound with a C₁₋₄ alkyl group or a C₁₋₅ acyl group, or protecting at least one pair of diols of the sulfoxide compound by cyclic ketalization, and trisulfidizing the modified or protected sulfoxide compound.

Production method (A) includes modifying at least one hydroxyl group of compound (10a) with a C₁₋₄ alkyl group or a C₁₋₅ acyl group, or protecting at least one pair of diols of compound (10a) by cyclic ketalization. Compound (10a) can be synthesized by the method described in Patent Literature 1. Production method (A) may include a step (Step A-1) of oxidizing a disulfide compound with an oxidizing agent to obtain a sulfoxide compound or a step (Step A-1') of oxidizing a thiol compound with an oxidizing agent to obtain a sulfoxide compound, a step (Step A-2) of allowing the obtained sulfoxide compound to react with a sulfur source to obtain compound (10a), and a step (Step A-3) of modifying the hydroxyl group of the obtained compound (10a) with a C₁₋₄ alkyl group or a C₁₋₅ acyl group, or protecting at least one pair of diols of the trisulfide compound by cyclic ketalization.

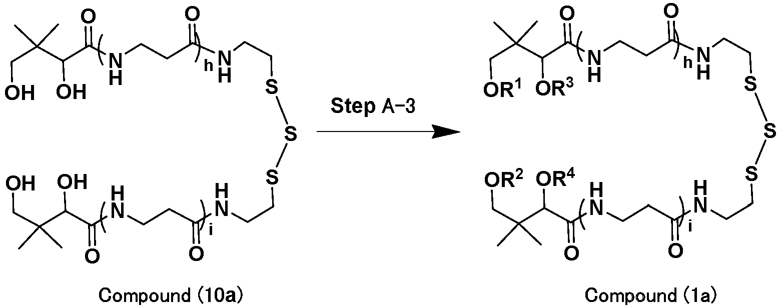

Production method (A), when h and i are 0, can further include the step shown in the following reaction scheme (I) to obtain compound (10a) in which h and i are 0.

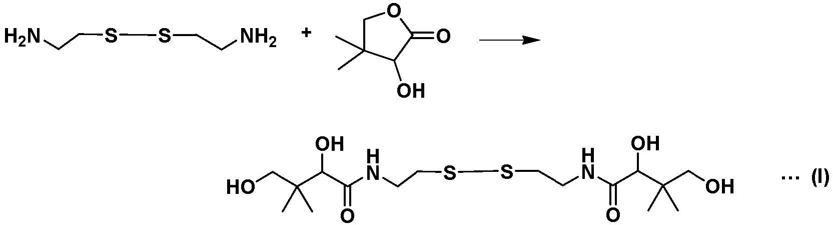

Production method (B-1) may include a step (Step B-1-1) of modifying at least one hydroxyl group of compound (11a) with a C₁₋₄ alkyl group or a C₁₋₅ acyl group, or protecting at least one pair of diols of compound (11a) by cyclic ketalization to obtain compound (12a), and a step (Step B-1-2) of trisulfidizing compound (12a). Step B-1-2 may be carried out according to the method described in Patent Literature 1. Step B-1-2 may include a step (Step B-1-2-1) of oxidizing compound (12a) with an oxidizing agent to obtain a sulfoxide compound, and a step (Step B-1-2-2) of allowing the obtained sulfoxide compound to react with a sulfur source to obtain compound (1). In addition, production method (B-1) may be a method in which Step B-1-2-1 is directly performed on compound (11a), and then Step B-1-1 and Step B-1-2-2 are performed in this order on the obtained sulfoxide compound to produce compound (1a). When h and i are 0, production method (B-1) can further include the step shown in the above reaction scheme (I) to obtain compound (11a) in which h and i are 0.

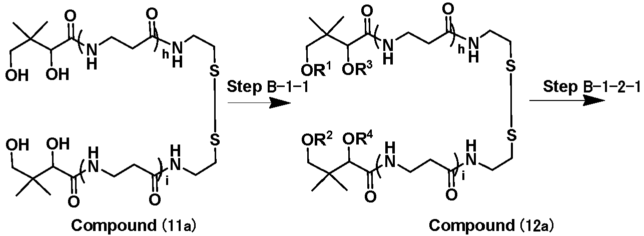

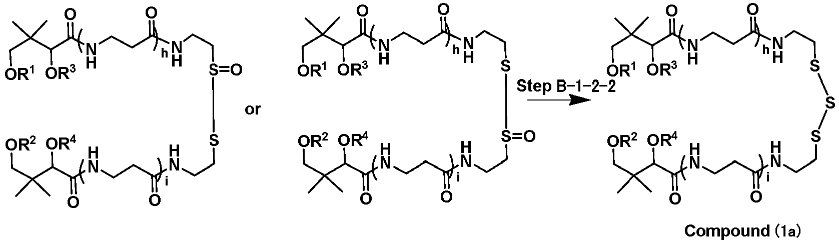

Production method (B-2) may include a step (Step B-2-1) of modifying at least one hydroxyl group of compound (13a) and compound (14a) with a C₁₋₄ alkyl group or a C₁₋₅ acyl group, or protecting at least one pair of diols of compound (13a) and compound (14a) by cyclic ketalization to obtain compound (15a) and compound (16a), and a step (Step B-2-2) of obtaining a trisulfide compound from compound (15a) and compound (16a). Step B-2-2 may be carried out according to the method described in Patent Literature 1. Step B-2-2 may include a step (Step B-2-2-1) of oxidizing compound (15a) and compound (16a) with an oxidizing agent to obtain a sulfoxide compound, and a step (Step B-2-2-2) of reacting the obtained sulfoxide compound with a sulfur source to obtain a trisulfide compound. In addition, production method (B-2) may be a method in which Step B-2-2-1 is directly performed on compound (13a) and compound (14a), and then Step B-2-1 and Step B-2-2-2 are performed in this order on the obtained sulfoxide compound to produce compound (1a).

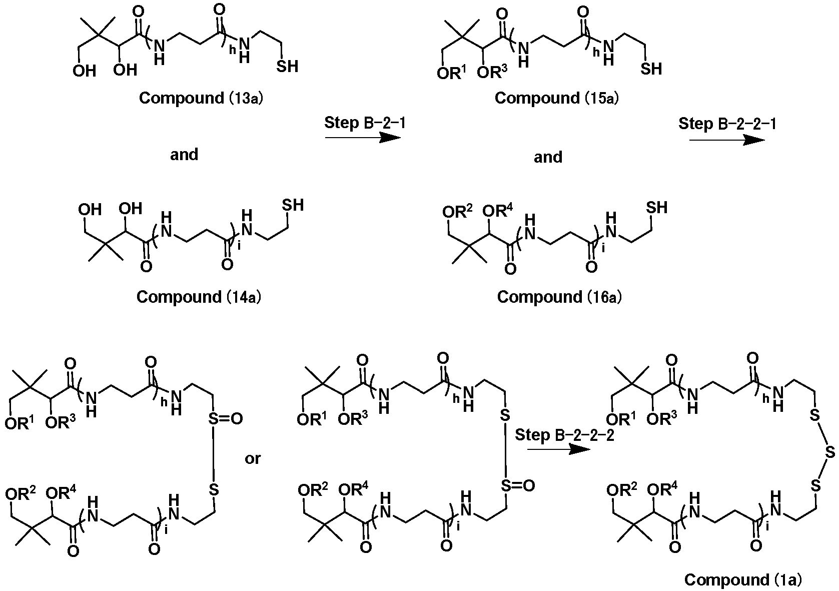

The solvent used in Steps A-1, A-1', B-1-2-1, and B-2-2-1 is one that dissolves the thiol compound, disulfide compound, and oxidizing agent and does not inhibit the oxidation reaction. Examples include water, aqueous sulfuric acid, aqueous ethanol, and aqueous acetonitrile, and preferably water. The amount of solvent used in Steps A-1, A-1', B-1-2-1, and B-2-2-1 may be 1 mL to 500 mL per 1 g of thiol compound or disulfide compound, and preferably 10 mL to 20 mL.

Examples of the oxidizing agent used in Steps A-1, A-1', B-1-2-1, and B-2-2-1 include potassium peroxymonosulfate (sold under the trade name Oxone^{®}), peracetic acid, hydrogen peroxide, and sodium periodate. Hydrogen peroxide may be used together with a catalytic amount of methyltrioxorhenium. From the viewpoint of safety and cost, potassium peroxymonosulfate is a preferred oxidizing agent. The amount of oxidizing agent used may be 0.8 to 2.0 equivalents per 1 equivalent of thiol compound or disulfide compound, and preferably 1.0 to 1.3 equivalents.

The reaction temperature in Steps A-1, A-1', B-1-2-1, and B-2-2-1 may be -20°C to 30°C, and preferably -5°C to 5°C.

The reaction time in Steps A-1, A-1', B-1-2-1, and B-2-2-1 may be 5 minutes to 24 hours or 10 minutes to 24 hours, and preferably 0.5 hours to 2 hours.

The solvent used in Steps A-2, B-1-2-2, and B-2-2-2 is one that dissolves the intermediate sulfoxide compound and does not inhibit the subsequent reaction. Examples include water, aqueous sulfuric acid, aqueous ethanol, and aqueous acetonitrile, and preferably water. The amount of solvent used in Steps A-2, B-1-2-2, and B-2-2-2 may be 1 mL to 500 mL per 1 g of sulfoxide compound, and preferably 10 mL to 20 mL.

Examples of the sulfur source used in Steps A-2, B-1-2-2, and B-2-2-2 include sodium sulfide, potassium sulfide, sodium hydrosulfide, potassium hydrosulfide, and hydrogen sulfide. The amount of sulfur source used may be 0.5 to 4.0 equivalents per 1 equivalent of sulfoxide compound, and preferably 0.9 to 1.2 equivalents.

The reaction temperature in Steps A-2, B-1-2-2, and B-2-2-2 may be -20°C to 30°C, and preferably -5°C to 25°C.

The reaction time in Steps A-2, B-1-2-2, and B-2-2-2 may be 10 minutes to 2 days, and preferably 0.5 hours to 2 hours.

Steps A-3, B-1-1, and B-2-1 may be carried out by methods well known in the art of synthetic organic chemistry. For example, alkylation of a hydroxyl group may be carried out by allowing compound (10a), compound (11a), or compound (13a) and compound (14a) to react with methylene chloride and an alkylating agent such as 2-dimethylaminoethyl chloride hydrochloride, or (2-iodoethoxy)triisopropylsilane in a solvent such as N,N-dimethylformamide, in the presence or absence of a base such as cesium carbonate or potassium carbonate. For example, esterification of a hydroxyl group may be carried out by allowing compound (10a), compound (11a), or compound (13a) and compound (14a) to react with acetic anhydride, acetyl chloride, benzoyl chloride, etc., in the presence of methylene chloride (dichloromethane, DCM) and a base such as pyridine, or triethylamine. For example, cyclic ketalization of a diol may be carried out by allowing compound (10a), compound (11a), or compound (13a) and compound (14a) to react with 2-methoxypropene, 2,2-dimethoxypropane, triphosgene, etc., in a solvent such as tetrahydrofuran, in the presence of (+)-10-camphorsulfonic acid, pyridine, or the like.

Each step included in production methods (A), (B-1), and (B-2) may be carried out in one pot without isolating the intermediate sulfoxide compound.

When each step included in production methods (A), (B-1), and (B-2) is carried out in one pot, examples of the reaction solvent include water, aqueous sulfuric acid, aqueous ethanol, and aqueous acetonitrile, and preferably water. The amount of solvent may be 1 mL to 500 mL per 1 g of thiol compound or disulfide compound, and preferably 10 mL to 20 mL. Examples of the oxidizing agent used include potassium peroxymonosulfate, peracetic acid, hydrogen peroxide (which may be used together with a catalytic amount of methyltrioxorhenium), and sodium periodate. The preferred oxidizing agent is potassium peroxymonosulfate. The amount of oxidizing agent used may be 0.8 to 2.0 equivalents per 1 equivalent of thiol compound or disulfide compound, and preferably 1.0 to 1.3 equivalents. Examples of the sulfur source used include sodium sulfide, potassium sulfide, sodium hydrosulfide, potassium hydrosulfide, and hydrogen sulfide. The amount of sulfur source used may be 0.5 to 4.0 equivalents per 1 equivalent of sulfoxide compound, and preferably 0.9 to 1.2 equivalents. The reaction temperature may be -20°C to 30°C, and preferably -5°C to 25°C. The reaction time may be 15 minutes to 2 days, and preferably 1 hour to 4 hours.

Among the compounds (1) according to one embodiment of the present invention, a compound in which either R¹⁰ or R¹¹ is a hydrogen atom may be produced by appropriately changing the starting material in the above production method for compound (1a). That is, by using compound (10) instead of compound (10a), compound (11) instead of compound (11a), or compound (13) and compound (14a) or compound (13a) and compound (14) instead of compound (13a) and compound (14a) as starting materials and carrying out the same reaction, the desired compound can be obtained.

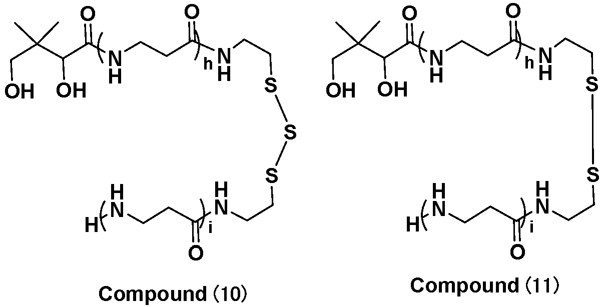

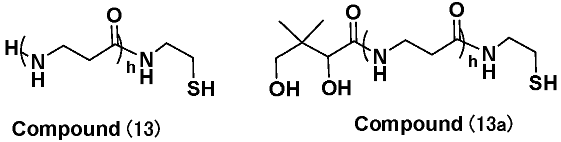

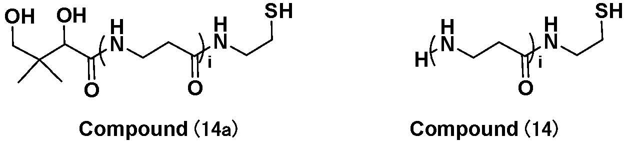

### Examples

The present invention will be described in detail below with reference to Examples, but the present invention is not limited to these Examples.

### Example 1: Preparation of Acetonide Derivative of Pantethine Trisulfide

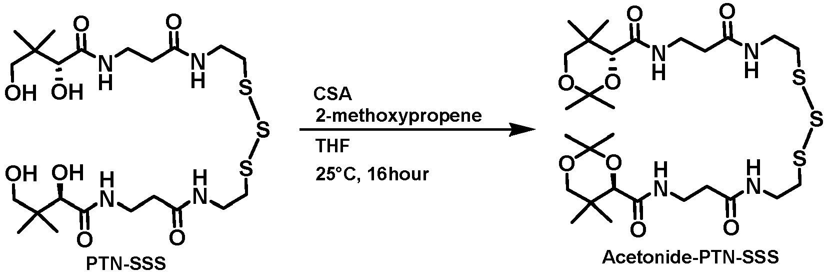

Pantethine trisulfide (PTN-SSS) (127 mg, 0.22 mmol) and tetrahydrofuran (THF) (3.8 mL, 33 v/w) were placed in a 10 mL test tube. After confirming that the contents of the flask were dissolved, 2-methoxypropene (0.20 mL, 2.16 mmol, 10 equivalents) and (+)-10-camphorsulfonic acid (CSA) (8.2 mg, 0.04 mmol, 0.2 equivalents) were added to the flask. The air in the flask was replaced with nitrogen, and the mixture was allowed to react at room temperature for 16 hours. After the reaction mixture was concentrated under reduced pressure, 1 mL of methanol was added to dissolve the residue. The mixture was purified by column chromatography (ODS column, mobile phase: water/acetonitrile). Finally, the mixture was concentrated under reduced pressure at an external temperature of 30°C and dried with an oil pump to give 97.8 mg (0.15 mmol, yield: 68%, HPLC purity: 95%) of the acetonide derivative of pantethine trisulfide (Acetonide-PTN-SSS) as a white solid. The ClogP of Acetonide-PTN-SSS is 2.74, logP is 1.56, and the molecular weight is 667. The ClogP of PTN-SSS is -1.99, and logP is -0.39.
¹H-NMR: (CDCl₃, 400 MHz) δ (ppm) = 7.04 (t, 2H, J = 6.0 Hz), 6.72 (t, 2H, J = 6.0 Hz), 4.08 (s, 2H), 3.73-3.48 (m, 10H), 3.28 (d, 2H, J = 11.2 Hz), 3.03 (t, 4H, J = 6.4 Hz), 2.49 (td, 4H, J = 6.4, 1.2 Hz), 1.47 (s, 6H), 1.43 (s, 6H), 1.03 (s, 6H), 0.97 (s, 6H).
ESI-TOF-MS: m/z 667.2889 ([M+H]⁺), calcd for [C_{2g}H₅₁N₄O₈S₃] 667.2869.

### Example 2: Preparation of Acetyl Derivative of Pantethine Trisulfide

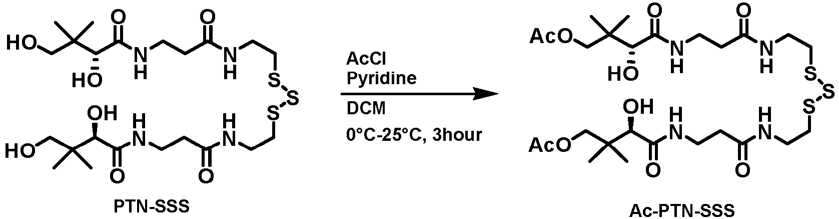

Pantethine trisulfide (PTN-SSS) (229 mg, 0.39 mmol) and methylene chloride (dichloromethane; DCM) (14 mL, 60 v/w) were added to a 25 mL round-bottom flask and cooled to 0°C. After addition of acetyl chloride (AcCl) (83 **µL,** 1.17 mmol, 3.0 equivalents), pyridine (95 **µL,** 1.17 mmol, 3.0 equivalents) was added. The air in the flask was replaced with nitrogen, and the mixture was allowed to react at room temperature for 3 hours. After addition of 4 mL of water at 0°C, a mixture of methylene chloride:methanol (10:1, 6 mL) was added, and the organic layer was extracted. This extraction was repeated three times, and the obtained organic layer was washed once with 6 mL of water. The washed organic layer was concentrated under reduced pressure at an external temperature of 30°C, dissolved in 2 mL of methylene chloride and a small amount of methanol, and purified by column chromatography (mobile phase: methylene chloride/methanol). Finally, the mixture was concentrated under reduced pressure at an external temperature of 30°C and dried with an oil pump to give 123 mg (0.18 mmol, yield: 47%, HPLC purity: 99%) of the acetyl derivative of pantethine trisulfide (Ac-PTN-SSS) as a white solid. The ClogP of Ac-PTN-SSS is -0.10, logP is 0.91, and the molecular weight is 671. The ClogP of PTN-SSS is -1.99, and logP is -0.39.
¹H-NMR: (CDCl₃, 400 MHz) δ (ppm) = 7.42 (br, 2H), 7.14 (br, 2H), 4.12 (d, 2H, J = 10.4 Hz), 3.92 (s, 2H), 3.84 (d, 2H, J = 11.2 Hz), 3.70-3.48 (m, 8H), 3.03 (t, 4H, J = 6.4 Hz), 2.49 (td, 4H, J = 5.6, 5.2 Hz), 2.09 (s, 6H), 1.03 (s, 6H), 0.95 (s, 6H).
ESI-TOF-MS: m/z 671.2481 ([M+H]⁺), calcd for [C₂₆H₄₇N₄O₁₀S₃] 671.2454.

The HPLC conditions in Examples 1 and 2 were as follows:
Detector: UV spectrophotometer (measurement wavelength: 220 nm)
Column: LiChrosorb RP-18 (Kanto Chemical, 4.0 × 250 mm, 5 µm)
Column temperature: constant temperature around 40°C
Mobile phase A: aqueous phosphoric acid solution (pH 3)
Mobile phase B: methanol
The mixing ratio of mobile phases A and B was changed as shown in Table 4 to control the concentration gradient.

**[Table 4]**

| Time after injection (min) | Mobile phase A (vol%) | Mobile phase B (vol%) |
|---|---|---|
| 0-3 | 70 | 30 |
| 3-20 | from 70 to 20 | from 30 to 80 |
| 20-25 | 20 | 80 |
| 25-26 | from 20 to 70 | from 80 to 30 |
| 26-35 | 70 | 30 |

Flow rate: 0.6 mL/min
Injection volume: 10 µL

### Example 3: Preparation of Compound 2

### Preparation of Compound 1

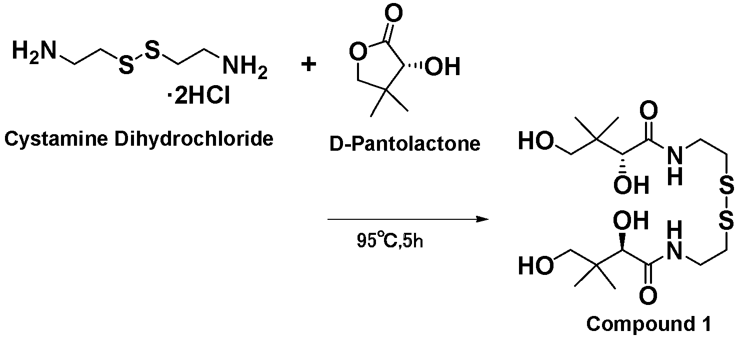

Cystamine dihydrochloride (5 g, 22.2 mmol) was dissolved in 5 mL of water (1 v/w), and 48% NaOH aq. (2.6 mL, 46.6 mmol, 2.1 equivalents) was added. Then, 50 mL of EtOH was added, and the inorganic salt was filtered off. The filtrate was concentrated under reduced pressure using an evaporator, and D-pantolactone (5.78 g, 44.4 mmol, 2.0 equivalents) was added to the concentrate, heated to 95°C, and allowed to react for 5 hours. After dissolving the reaction mixture with 2 mL of EtOH, the mixture was purified by column chromatography (mobile phase A: ethyl acetate, mobile phase B: methanol) to give 8.51 g (20.6 mmol, yield: 93%, HPLC purity: 99%) of Compound 12.
¹H-NMR: (D₂O, 400 MHz) δ (ppm) = 3.95 (s, 2H), 3.53 (dd, 4H, J = 6.4, 6.0 Hz), 3.48 (d, 2H, J = 11.2 Hz), 3.35 (d, 4H, J = 11.2 Hz), 2.85 (dd, 4H, J = 6.4, 6.4 Hz), 0.90 (s, 6H), 0.86 (s, 6H).

The HPLC conditions were the same as in Examples 1 and 2, except that the detector measurement wavelength was 210 nm.

### Preparation of Compound 2

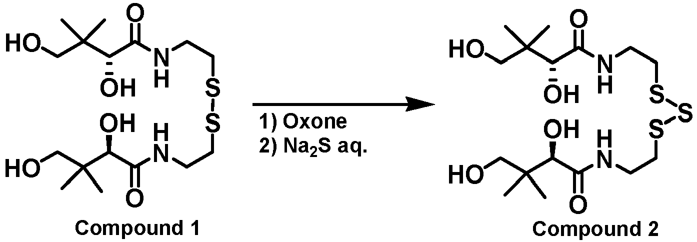

Compound 1 (252 mg, 611 µmol) and 3 mL of water (12 v/w) were placed in a reaction vessel and cooled to an internal temperature of 1°C. Oxone^{®} (208 mg, 672 µmol, 1.1 equivalents) was added and the mixture was allowed to react for 90 minutes. Then, a sodium sulfide solution prepared by dissolving sodium sulfide nonahydrate (147 mg, 611 µmol, 1.0 equivalent) in 1.5 mL of water was added dropwise, and the mixture was allowed to react for about 1 hour. After addition of 6 mL of ethanol, the inorganic salt was filtered off and washed with 2 mL of ethanol. The filtrate was concentrated under reduced pressure using an evaporator, and the concentrate was purified by ODS column chromatography (mobile phase A: 0.1% aqueous formic acid, mobile phase B: ethanol) to give 130 mg (259 µmol, yield: 48%, HPLC purity: 99%) of Compound 2.
¹H-NMR: (D₂O, 400 MHz) δ (ppm) = 3.97 (s, 2H), 3.62 (dd, 4H, J = 6.4, 6.0 Hz), 3.49 (d, 2H, J = 10.8 Hz), 3.37 (d, 4H, J = 11.2 Hz), 3.11 (dd, 4H, J = 6.4, 6.4 Hz), 0.92 (s, 6H), 0.88 (s, 6H).
ESI-TOF-MS: m/z 445.1544 ([M+H]⁺), calcd for [C₁₆H₃₃N₂O₆S₃] 445.1501.

The HPLC conditions were the same as in Examples 1 and 2, except that the detector measurement wavelength was 210 nm.

### Example 4: Preparation of Compound 4

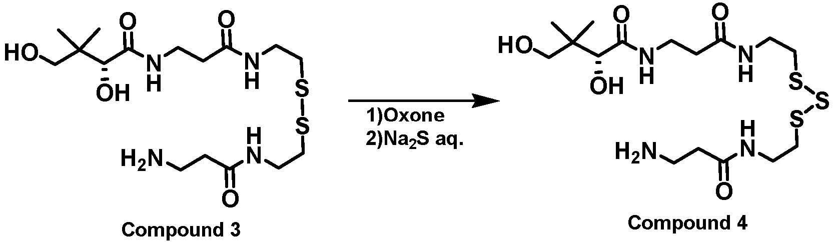

Compound 3 (1.01 g, 2.38 mmol) and 13 mL of water (13 v/w) were placed in a reaction vessel and cooled to an internal temperature of 1°C. Oxone^{®} (892 mg, 2.62 mmol, 1.1 equivalents) was added and the mixture was allowed to react for 90 minutes. Then, a sodium sulfide solution prepared by dissolving sodium sulfide nonahydrate (571 mg, 2.38 mmol, 1.0 equivalent) in 5.6 mL of water was added dropwise, and the mixture was allowed to react for about 1 hour. The reaction mixture was purified by ODS column chromatography (mobile phase A: aqueous formic acid, mobile phase B: ethanol) to give 372 mg (0.82 mmol, yield: 34%, HPLC purity: 96%) of Compound 4.
¹H-NMR: (D₂O, 400 MHz) δ (ppm) = 3.89 (s, 1H), 3.58-3.36 (m, 8H), 3.18 (t, 2H, J = 6.9 Hz), 2.98 (dt, 4H, J = 6.9, 6.4 Hz), 2.60 (t, 2H, J = 6.9 Hz), 2.42 (t, 2H, J = 6.4 Hz), 1.08 (dd, 2H, J = 6.9, 7.3 Hz), 0.83 (s, 3H), 0.79 (s, 3H).
ESI-TOF-MS: m/z 457.1653 ([M+H]⁺), calcd for [C₁₆H₃₃N₄O₅S₃] 457.1613.

The HPLC conditions were as follows:
Detector: UV spectrophotometer (measurement wavelength: 220 nm)
Column: Meteoric Core C18 (YMC, 4.6 × 150 mm, 2.7 µm)
Column temperature: constant temperature around 40°C
Mobile phase A: aqueous phosphoric acid solution (pH 3)
Mobile phase B: methanol
The mixing ratio of mobile phases A and B was changed as follows to control the concentration gradient:

**[Table 5]**

| Time after injection (min) | Mobile phase A (vol%) | Mobile phase B (vol%) |
|---|---|---|
| 0-3 | 100 | 0 |
| 3-20 | from 100 to 50 | from 0 to 50 |
| 20-25 | 50 | 50 |
| 25-26 | from 50 to 100 | from 50 to 0 |
| 26-35 | 100 | 0 |

Flow rate: 0.4 mL/min
Injection volume: 10 µL

### Example 5: Preparation of Compound 5

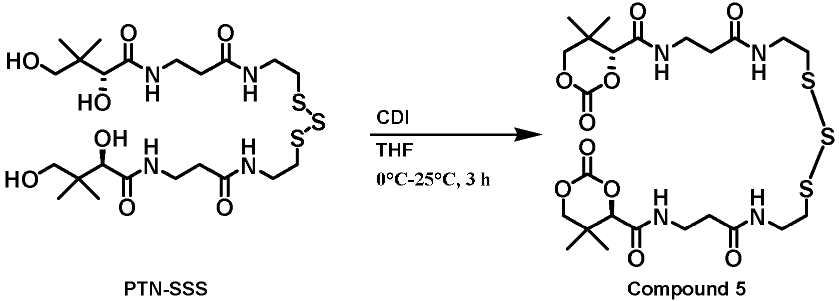

Pantethine trisulfide (PTN-SSS) (117 mg, 0.20 mmol) and 6 mL of methylene chloride (60 v/w) were placed in a 10 mL round-bottom flask, and carbonyldiimidazole (CDI) (168 mg, 1.03 mmol, 5.2 equivalents) was added. The air in the flask was replaced with nitrogen, and the mixture was allowed to react at room temperature for 3 hours. After the reaction mixture was concentrated under reduced pressure at an external temperature of 30°C and purified by column chromatography (mobile phase: water/methanol). Finally, after further concentration under reduced pressure at 30°C, the residue was extracted with ethyl acetate using liquid-liquid extraction, and again concentrated under reduced pressure at 30°C. The residue was dried with an oil pump to give 2.7 mg (0.04 mmol, yield: 2%, HPLC purity: 99%) of compound 5 as a brown oil.
¹H-NMR: (MeOH-d₄, 400 MHz) δ (ppm) = 4.72 (s, 2H), 3.82-3.68 (m, 4H), 3.51-3.37 (m, 8H), 2.98 (dd, 4H, J = 6.9, 6.4 Hz), 2.51 (t, 4H, J = 6.86 Hz), 1.02 (s, 6H), 0.99 (s, 6H).
ESI-TOF-MS: m/z 639.1821 ([M+H]⁺), calcd for [C₂₄H₃₉N₄O₁₀S₃] 639.1828.

The HPLC conditions were the same as in Example 4.

### Test Example 1

The hydrogen sulfide trapping ability (hydrogen sulfide elimination effect) of the compound represented by Formula (1) was evaluated by the methylene blue spectrophotometric method.

### Preparation of H₂S standard solution

A solution of sodium sulfide nonahydrate (Na₂S·9H₂O) was prepared by dissolving it in purified water to prepare a 50 µmol/L sodium sulfide nonahydrate solution, which was used as the H₂S standard solution.

### Preparation of p-Aminodimethylaniline (DPDA) solution

p-Aminodimethylaniline was dissolved in 10N sulfuric acid to prepare a 1 mmol/L DPDA solution.

### Preparation of Ferric chloride (III) solution

Ferric chloride (III) hexahydrate was dissolved in 1% sulfuric acid to prepare a 3 mmol/L FeCl₃ solution.

### Preparation of trisulfide compound solution

The trisulfide compound was dissolved in 25 mmol/L phosphate buffer (pH 7, hereinafter referred to as "PBS") to prepare a solution of the trisulfide compound at a concentration of 50 µmol/L.

### Test Method

10 mL (0.5 µmol, reference) of the H₂S standard solution was added to a stoppered test tube and stirred. To this solution, 1 mL (0.5 µmol, 1 equivalent) of PBS (blank) or the trisulfide compound solution was added and stirred for a predetermined time (0 min, 5 min, 15 min, or 30 min). After stirring, 1 mL (1 µmol, 2 equivalents) of DPDA solution and 1 mL (3 µmol, 6 equivalents) of FeCl₃ solution were added and further stirred. After the absorbance of the solution no longer changed (i.e., after the coloration due to methylene blue formation was complete), the absorbance of the solution (wavelength: 668 nm) was measured. This test was conducted at room temperature.

### Test Results

The results are shown in Figure 1. In Figure 1, the horizontal axis "stirring time" indicates the stirring time of the mixture of the H₂S standard solution and PBS (blank) or the trisulfide compound solution before the addition of the color-developing agents (DPDA solution and FeCl₃ solution). It was confirmed that the longer the contact time (reaction time) between H₂S and the trisulfide compound, the lower the absorbance compared to the blank. This is presumed to be because H₂S reacts with the trisulfide compound and is consumed, resulting in a decrease in the amount of methylene blue produced compared to the blank. From the above, it was confirmed that the compound represented by Formula (1) or a salt thereof has hydrogen sulfide trapping ability (hydrogen sulfide elimination effect).

### Test Example 2

Pantethine trisulfide (PTN-SSS) (6.7 mg), Acetonide-PTN-SSS (Example 1) (5.3 mg), and Ac-PTN-SSS (Example 2) (5.3 mg) were each dissolved in 10 mL of purified water. Then, 10 mL of octanol was added to each aqueous solution and shaken for 1 minute. 2 mL each was taken from the aqueous layer and the octanol layer, centrifuged, and allowed to stand for 1 hour. 1 mL was precisely measured from the standing solution, 2-propanol was added, and the volume was made up to 10 mL. The concentration of each compound in each layer was calculated from the HPLC analysis results, and the partition coefficient (logP) was calculated. (logP = log₁₀ (concentration in octanol layer / concentration in aqueous layer))

The results are shown in Table 6.

**[Table 6]**

| Compound | PTN-SSS | Acetonide-PTN-SSS (Example 1) | Ac-PTN-SSS (Example 2) |
|---|---|---|---|
| Partition coefficient (logP) | -0.39 | 1.56 | 0.91 |

The value of the partition coefficient increased in the order of PTN-SSS < Ac-PTN-SSS < Acetonide-PTN-SSS. This result is presumed to be due to the protection of the hydroxyl group of PTN-SSS. Since Acetonide-PTN-SSS and Ac-PTN-SSS have higher partition coefficient values than PTN-SSS, it was revealed that the compound represented by Formula (1) or a salt thereof has improved lipophilicity compared to PTN-SSS.

## Claims

1. A compound represented by the Formula (1) or a salt thereof: 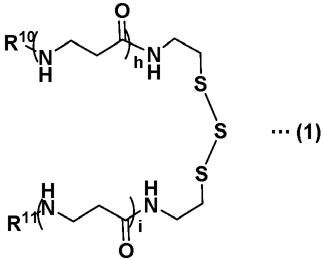
wherein R¹⁰ is a hydrogen atom or a group represented by 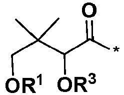 wherein * is a bond, and
R¹¹ is a hydrogen atom or a group represented by 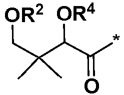 wherein * is a bond, with a proviso that R¹⁰ and R¹¹ are not hydrogen atoms at the same time,
wherein R¹, R², R³, and R⁴ are each independently a hydrogen atom, a C₁₋₉ alkyl group, or a C₁₋₅ acyl group, and R¹ and R³ may together form a group represented by the Formula (2): 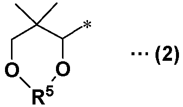 wherein R⁵ is a methylene group optionally having a C₁₋₄ alkyl group as a substituent, and * is a bond, or a carbonyl group, R² and R⁴ may together form a group represented by the Formula (2): 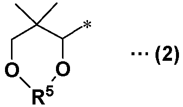 wherein R⁵ is a methylene group optionally having a C₁₋₄ alkyl group as a substituent, and * is a bond, or a carbonyl group, with a proviso that at least one of R¹, R², R³, or R⁴ is a C₁₋₉ alkyl group or a C₁₋₅ acyl group, and h and i are each independently 0 or 1.

2. The compound or salt thereof according to claim 1, wherein R¹⁰ is a group represented by 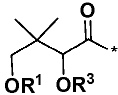 wherein * is a bond, and R¹¹ is a group represented by 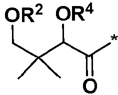 wherein * is a bond.

3. The compound or salt thereof according to claim 1, wherein R¹, R², R³, and R⁴ are each independently a hydrogen atom or a C₁₋₄ alkyl group, and at least one ofR¹, R², R³, or R⁴ is a C₁₋₄ alkyl group.

4. The compound or salt thereof according to claim 1, wherein R¹, R², R³, and R⁴ are each independently a hydrogen atom or a C₁₋₅ acyl group, and at least one ofR¹, R², R³, or R⁴ is a C₁₋₅ acyl group.

5. The compound or salt thereof according to claim 1, wherein R¹ and R³ together form a group represented by Formula (2) or a carbonyl group, and R² and R⁴ together form a group represented by Formula (2) or a carbonyl group.

6. The compound or salt thereof according to claim 2, wherein R¹, R², R³, and R⁴ are each independently a hydrogen atom or a C₁₋₄ alkyl group, and at least one ofR¹, R², R³, or R⁴ is a C₁₋₄ alkyl group.

7. The compound or salt thereof according to claim 2, wherein R¹, R², R³, and R⁴ are each independently a hydrogen atom or a C₁₋₅ acyl group, and at least one ofR¹, R², R³, or R⁴ is a C₁₋₅ acyl group.

8. The compound or salt thereof according to claim 2, wherein R¹ and R³ together form a group represented by Formula (2) or a carbonyl group, and R² and R⁴ together form a group represented by Formula (2) or a carbonyl group.

9. The compound or salt thereof according to any one of claims 1 to 8, wherein h and i are 0.
